# EUROPEAN PATENT APPLICATION

(11) **EP 3 289 864 A1**
(43) Date of publication of application: **07.03.2018**
(21) Application number: 16786662.3
(22) Date of filing: 08.04.2016
(51) Int. Cl.: A01K 61/00, A01K 63/02, C07C 255/35

(54) **COMPOSITION FOR INDUCING HYPOMETABOLISM, METHOD FOR INDUCING HYPOMETABOLISM, AND FISH TRANSPORTATION METHOD USING TECHNIQUES THEREOF**

(30) Priority: 30.04.2015 KR 20150061860
(71) Applicant: University Industry Foundation, Yonsei University Wonju Campus, Gangwon-do 26493 (KR)
(72) Inventor: CHOI, Inho, Wonju-si, Gangwon-do 26435 (KR); CHUNG, Chan Moon, Wonju-si, Gangwon-do 26399 (KR); CHANG, Man, Seongnam-si, Gyeonggi-do 13583 (KR); PARK, Min Jae, Seoul 02086 (KR); SO, Hye kyung, Seoul 06310 (KR)
(74) Representative: Ström & Gulliksson AB
(86) International application number: PCT/KR2016/003720
(87) International publication number: WO 2016/175476

(57) **Abstract**

The present invention relates to a composition for inducing hypometabolism of fish, a method for inducing hypometabolism, and a fish transportation method using techniques thereof. More particularly, a method for inducing long-term hypometabolism of fish of the present invention enables the ventilation frequency of fish to decrease by treating the fish with T1AM, which is a hypometabolic-inducing substance and enables the ventilation frequency to be recovered to an original state when T1AM is removed. In addition, although the oxygen consumption rate decreases and the survival time increases as the ventilation frequency decreases, there is no change in the expression profile of chaperone which occurs due to stress, such as long-term oxygen deficiency, and a decrease in ventilation frequency by T1AM exhibits the same effect as a case of decreasing water temperature by 10°C. Therefore, by means of the technique, it is possible to provide a fish transportation method which is stable and economical while preventing deterioration of quality of fish meat.

## Description

### [Technical Field]

The present invention relates to a composition for inducing hypometabolism which induces hypometabolism by treating fish with a hypometabolism-inducing substance, a method for inducing hypometabolism, and a fish transportation method using techniques thereof.

### [Background Art]

3-iodothyronamine (T1AM) is a derivative of thyroid hormones T3 and T4 and a hypometabolism-inducing substance that may be generated in the body. It has been found that a pico mole of T1Am is present in most of the rodent tissue samples (brain, liver, heart, kidney, muscle, etc.) and the human blood (Zucchi R et al., 2006). In addition, 3-iodotronamine is a synthesizable substance and a preparing method thereof is disclosed in U.S. Patent Registration Nos. 6,979,750 and 7,321,065 and Korean Patent Registration No. 1,112,731, which is a prior patent of the inventor of the present application, and the 3-iodotronamine can be mass-produced to be easily used for industrial use.

Meanwhile, according to previous researches, it has been reported that when T1AM was administered intraperitoneally to mice (e.g., 50 mg/kg), the oxygen consumption rate and the body temperature start to decrease within several minutes, the body temperature decreases to 28 to 30°C after 1 hour 30 minutes to 2 hours after administration, and the body temperature is recovered to a normal temperature after 5 to 6 hours (Ju H et al., 2011). In addition, recently, according to *in vitro* experimental results, it has been found that when the muscle cells C2C12 are treated with T1AM (75 µM) for 6 hours, the oxygen consumption rate of the cells is decreased up to 60% compared to the control (Ju H et al., 2015). Based on these results, it can be hypothesized that T1AM will lower the metabolic rate (oxygen consumption rate) in other vertebrates as well as mammals (mice).

Fish are poikilothermic vertebrates, and phylogenetically correspond to the first classification of vertebrates. Accordingly, if a hypometabolism-inducing substance such as T1AM can lower the metabolic rate of the fish, it is presumed that amphibians, reptiles and algae corresponding to the middle position of vertebrates may also obtain the same result. However, the efficacy of the hypometabolism-inducing substance, T1AM, in fish, such as whether T1AM induces hypometabolism of fish and whether the metabolic rate is recovered when T1AM is removed, has not been found.

Except for a few species, the fish survive only in water, and thus, their growth and survival rates extremely vary depending on water quality and a water temperature. Accordingly, in order to increase the survival rate of the fish, a method for decreasing metabolism of fish by controlling a water temperature, such as Korean Patent Registration No. 232,408 which uses a method of cooling a temperature of sea water and Korean Patent Registration No. 740,457 which uses a stepwise temperature control of sea water is the most known. However, since such a water temperature control system can not be free from stress of the fish received due to a decrease in water temperature, deterioration of meat quality or a risk of perish are still involved, and cost burden is incurred by installation of the water temperature control system.

In addition, in the fish transportation method, there are an anesthetic transportation method, an electric shock transportation method, and the like such as Korean Patent Registration No. 1,208,741 using a natural anesthetic composition, but it is difficult to widely commercialize the methods due to safety and quality deterioration. There is an artificial hibernation method using hibernation induction, such as Japanese Patent Application Laid-Open No. 1999-220974, but since a similar hibernation inducing substance needs to be obtained from the serum of a hibernating animal and an awakening substance for awakening needs to be separately supplied, it is difficult to industrially commercialize the method.

Therefore, since inducing substances which can be mass-produced are used to induce the highest natural physiological regulation such as similar hibernation or a hypometabolic state, the fish survive for a long time without being stressed to be transported in a live fish state without damage of the quality. In addition, since a separate water temperature control device is not required, an economical fish transport technology that may decrease costs and is industrially valuable is required.

### [Prior Art]

### [Patent Document]

(Patent Document 1) US Patent Registration No. 6,979,750
(Patent Document 2) US Patent Registration No. 7,321,065
(Patent Document 3) Korean Patent Registration No. 1,112,731
(Patent Document 4) Korean Patent Registration No. 232,408
(Patent Document 5) Korean Patent Registration No. 740,457
(Patent Document 6) Korean Patent Registration No. 1,208,741
(Patent Document 7) Japanese Patent Laid-Open Publication No. 1999-220974

### [Non-Patent Document]

(Non-Patent Document 1) Zucchi R, Chiellini G, Scanlan TS, and Grand DK (2006) Trace amine-associated receptors and their ligands. Br J Pharmacol. 149:967-978.
(Non-Patent Document 2) Ju H, So H, Ha K, Park K. Lee J-W, Chung C-M, and Choi I. (2011) Sustained torpidity following multi-dose administration of 3-iodothyronamine in mice. Journal of Cellular Physiology 226:853-858.

### [Disclosure]

### [Technical Problem]

An object of the present invention is to provide a composition for inducing hypometabolism of fish by a hypometabolism-inducing substance which induces hypometabolism of the fish by treating a hypometabolism-inducing substance such as 3-iodothyronamine (T1AM) and recovers a metabolic rate by removing the hypometabolism-inducing substance, a method for inducing hypometabolism, and a fish transportation method using techniques thereof.

### [Technical Solution]

In order to achieve the object, an exemplary embodiment of the present invention provides a composition for inducing hypometabolism of fish by a hypometabolism-inducing substance, a method for inducing hypometabolism, and a fish transportation method using techniques thereof, by confirming a ventilation frequency of fish, an oxygen consumption rate, a survival time, and a change in expression profile of stress-responsive protein according to treatment and removal of 3-iodothyronamine (T1AM) to first find a hypometabolism induction effect of T1AM in the fish.

### [Advantageous Effects]

According to the present invention, in the method for inducing hypometabolism of fish by the hypometabolism-inducing substance, the ventilation frequency is decreased by the treatment of the hypometabolism-inducing substance such as T1 AM and the ventilation frequency is recovered to an original state when the hypometabolism-inducing substance is removed, thereby exhibiting the same effect as lowering the water temperature without controlling the water temperature. In addition, as the ventilation frequency is decreased, the consumption rate of oxygen is decreased, a survival time is increased, but there is no change in expression profile of chaperone generated due to stress such as long-term oxygen deficiency. As a result, the long-term hypometabolism of the fish by the hypometabolism-inducing substance of the present invention does not act as stress and may be used for fish transportation as a method for inducing hypometabolism of fish which may be recovered to a normal state with the removal of the hypometabolism-inducing substance.

### [Description of Drawings]

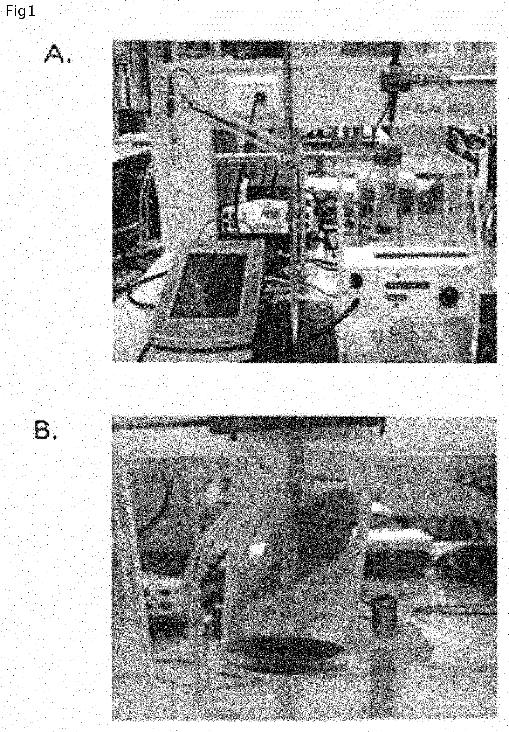

FIG. 1 is a photograph of a measuring device (A) for measuring the ventilation frequency of *Hypostomus plecostomus* and a state (B) experimented by adding *Hypostomus plecostomus* into a tube containing a solution using the same.
FIG. 2 is a graph illustrating a change in ventilation frequency of *Hypostomus plecostomus* according to a concentration of TIAM.
FIG. 3 is a graph illustrating a change (A) (n=6) in ventilation frequency of *Hypostomus plecostomus* before and after TIAM is administered at a concentration of 0.005% and a change (B) (n=5) in long-term ventilation frequency of *Hypostomus plecostomus* which is recovered again by removing the TIAM.
FIG. 4 is a graph illustrating a change in ventilation frequency of *Hypostomus plecostomus* in a vehicle solution.
FIG. 5 is a graph (n=6) illustrating a change in ventilation frequency of *Hypostomus plecostomus* according to a water temperature.

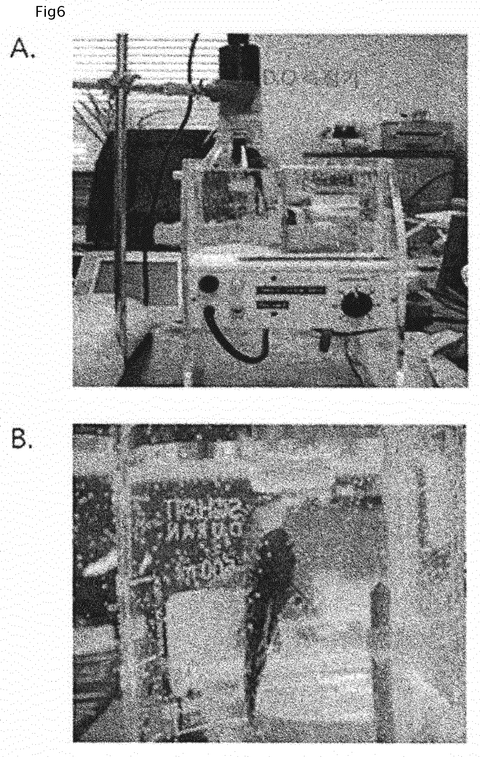

FIG. 6 is a photograph of a measuring device (A) for measuring the oxygen consumption rate of *Hypostomus plecostomus,* and a state (B) experimented by adding *Hypostomus plecostomus* into a sealed glass bottle containing a solution using the same.
FIG. 7 is a comparative graph illustrating changes in dissolved oxygen (A) and ventilation frequency (B) measured by adding *Hypostomus plecostomus* into a sealed glass bottle containing a 0.005% TIAM solution or a control solution (a vehicle solution) (●: control, ○: TIAM).
FIG. 8 is a comparative graph illustrating changes in oxygen consumption rate (A) and ventilation frequency (B) measured by adding *Hypostomus plecostomus* into a sealed glass bottle containing a 0.005% TIAM solution or a control solution (a vehicle solution) (●: control, ○: TIAM) [*: it means that there is a significant difference between two groups (a TIAM solution and a control (vehicle solution) (independent paired t-test, P < 0.05)].
FIG. 9 illustrates expression levels of HSP90 and α(β-crystallin with respect to individuals over time after administration of TIAM (Con: 0 minute (before administration), 400T: after 400 minutes, 800T: after 800 minutes, 1200T: after 1200 minutes), individuals removed after administration (1200R: individuals recovered from a hypometabolic state induced by removing TIAM from 600 minutes to 1200 minutes after administration of TIAM), and individuals to which TIAM is administered (1200C: individuals in the vehicle solution up to 1200 minutes without TIAM administration) (n=4).

### [Modes of the Invention]

The present invention relates to a method for inducing hypometabolism of fish which is performed by adding the fish into a solution containing a hypometabolism-inducing substance selected from the group consisting of 3-iodothyronamine (T1AM), [D-Ala2,D-Leu5] enkephalin (DADLE), 5'-adenosine monophosphate (5'-AMP), and hydrogen sulfide (H₂S) or administering a hypometabolism-inducing substance selected from the group consisting of 3-iodothyronamine (T1AM), [D-Ala2,D-Leu5] enkephalin (DADLE), 5'-adenosine monophosphate (5'-AMP), and hydrogen sulfide (H₂S) in a solution containing fish.

In one embodiment of the present invention, the hypometabolism-inducing substance may be more particularly 3-iodothyronamine (T1AM).

In one embodiment of the present invention, when the hypometabolism-inducing substance is 3-iodothyronamine (T1AM), T1AM may be contained at a concentration of 0.002% (w/v) or more, particularly 0.002 to 0.01% (w/v), more particularly 0.003 to 0.0095% (w/v), and much more particularly 0.004 to 0.0085% (w/v), and much more particularly 0.005 to 0.0075% (w/v) with respect to the entire solution. However, the concentration of T1AM in the entire solution may be controlled by the species or the weight of an individual, and is not necessarily limited to the above range. When the concentration of T1AM is 0.002% (w/v) or less, stable hypometabolism may not be induced because the ventilation frequency does not show the lowest value even after 15 hours or more, and when the concentration of T1AM exceeds 0.01% (w/v), the lowest ventilation frequency may be very rapidly induced and thus, the fish may not fully comply with the low ventilation frequency.

In one embodiment of the invention, the hypometabolic state may be maintained for 1 hour or more, 2 hours or more, 3 hours or more, 4 hours or more, 5 hours or more, 6 hours or more, 7 hours or more, 8 hours or more, 9 hours or more, and 10 hours or more, more particularly 10 to 20 hours, and much more particularly about 20 hours. However, a desired hypometabolism induction time is not limited thereto and may be controlled by adjusting the concentration of the inducing substance.

In one embodiment of the present invention, the hypometabolism of fish may be recovered by removing the hypometabolism-inducing substance. The removal of the hypometabolism-inducing substance may be performed by, for example, a method of continuously injecting a vehicle solution at a predetermined speed, but the removal method is not limited thereto.

In one embodiment of the present invention, feed supply before inducing hypometabolism may be stopped, and this is to prevent diseases such as indigestion or enteritis which may occur in the hypometabolic state.

In one embodiment of the present invention, the induction of hypometabolism in the fish is performed under a constant temperature condition, which means maintaining a water temperature suitable for growth of fish species.

The present invention relates a fish transportation method using a hypometabolism-inducing substance including: a) adding fish into a solution containing a hypometabolism-inducing substance selected from the group consisting of 3-iodothyronamine (T1AM), [D-Ala2,D-Leu5] enkephalin (DADLE), 5'-adenosine monophosphate (5'-AMP), and hydrogen sulfide (H₂S); b) moving the fish in the solution in step a) to a destination; and c) recovering a hypometabolic state of the fish by removing the hypometabolism-inducing substance from the solution.

In one embodiment of the present invention, the hypometabolism-inducing substance for the fish transportation may be more particularly 3-iodothyronamine (T1AM).

In one embodiment of the present invention, when the hypometabolism-inducing substance for the fish transportation is 3-iodothyronamine (T1AM), T1AM may be contained at a concentration of 0.002% (w/v) or more, particularly 0.002 to 0.01% (w/v), more particularly 0.003 to 0.0095% (w/v), and much more particularly 0.004 to 0.0085% (w/v), and much more particularly 0.005 to 0.0075% (w/v) with respect to the entire solution. However, the concentration of T1AM in the entire solution may be controlled by the species or the weight of an individual, and is not necessarily limited to the above range. When the concentration of T1AM is 0.002% (w/v) or less, stable hypometabolism may not be induced because the ventilation frequency does not show the lowest value even after 20 hours, and when the concentration of T1AM exceeds 0.01% (w/v), the lowest ventilation frequency may be very rapidly induced and thus, the fish may not fully comply with the low ventilation frequency.

In one embodiment of the present invention, the induction of hypometabolism in the fish for the fish transportation is performed under a constant temperature condition, which means maintaining a water temperature suitable for growth of fish species.

In one embodiment of the present invention, the fish hypometabolic state for the fish transportation may involve air injection for long-term transportation.

The present invention relates to a composition for inducing hypometabolism of fish, containing a hypometabolism-inducing substance selected from the group consisting of 3-iodothyronamine (T1AM), D-Ala2,D-Leu5] enkephalin (DADLE), 5'-adenosine monophosphate (5'-AMP), and hydrogen sulfide (H₂S); and a solvent.

In one embodiment of the present invention, the hypometabolism-inducing substance may be 3-iodothyronamine (T1AM). In this case, the T1AM may be contained at a concentration of 0.002% (w/v) or more, particularly 0.002 to 0.01% (w/v), more particularly 0.003 to 0.0095% (w/v), and much more particularly 0.004 to 0.0085% (w/v), and much more particularly 0.005 to 0.0075% (w/v) with respect to the entire solution. However, the concentration of T1AM in the entire solution may be controlled by the species or the weight of an individual, and is not necessarily limited to the above range. When the concentration of T1AM is 0.002% (w/v) or less, stable hypometabolism may not be induced because the ventilation frequency does not show the lowest value even after 20 hours, and when the concentration of T1AM exceeds 0.01% (w/v), the lowest ventilation frequency may be very rapidly induced and thus, the fish may not fully comply with the low ventilation frequency.

In one embodiment of the present invention, the solvent for the composition for inducing hypometabolism of fish may be island water or seawater. The island water means all kinds of water or solvents suitable for the growth of fish in fresh water.

Hereinafter, the present invention will be described in more detail through Experimental Examples according to the present invention, but the scope of the present invention is not limited to Experimental Examples to be described below and the like.

### [Experimental Examples]

### ∘ Statistical analysis

All values corresponding to the measurement result were represented by mean ± SEM. A group differences for an oxygen consumption rate and a survival time were verified by an independent pair's t-test. Statistical analysis was performed using SPSS/PC+, and significance was determined at P = 0.05.

### ∘ Experimental animal and Management

All Experimental Examples used *Hypostomus plecostomus* without distinction of gender. Before the experiment, all individuals were maintained in a water bath temperature range of 25 ± 0.5°C, pH to 7.0, and 12:12 LD (light/dark) conditions. The individuals were fed at ten in the morning every day and fasted from 24 hours before the experiment.

### ∘ T1AM (3-iodothyronamine)

3-iodothyronamine (T1AM) used in all Experimental Examples was a substance produced by organic synthesis (Korean Patent Registration No. 1,112,731) and the suitability of the material properties was determined by NMR analysis and animal efficacy experiments.

### Experimental Example 1: Change in ventilation frequency according to T1AM concentration

Before the experiment, the body weight of the individual was measured with an electronic balance (AFX-200G, Shimadzu, JP). The experiment was performed using water (pH 6.5 to 7.5, 25°C) stored for 48 hours in a syringe tube (KOVAX-SYRINGE; 21GX1, Seoul, KR) containing individuals. An air pump is connected to the lower side of the tube to inject a small amount of air, and the temperature of water was measured by using a thermocouple thermometer (Cole-Parmer, Vermon Hills, IL). The water temperature (25°C) of the tube was kept constantly by making the water temperature constant in a single tissue bath (harvard).

The T1AM was dissolved and used in dimethyl sulfoxide (DMSO; SIGMA, Missouri, US) and the amount and concentration of T1AM were determined according to a weight of the individual and a water volume. An individual stabilization time in the tube was maintained for 400 minutes before TIAM administration, and a gill ventilation frequency of the individual was observed and recorded every 30 minutes at concentrations of 0.000%, 0.002%, 0.003%, 0.005%, and 0.0075% (w/v).

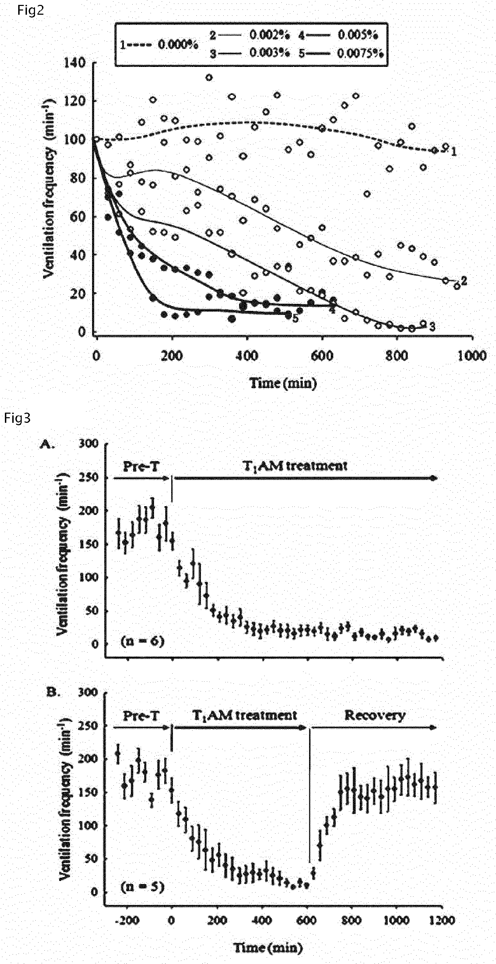

As the result of comparing a change in ventilation frequency in *Hypostomus plecostomus* according to T1AM concentrations of 0.000%, 0.002%, 0.003%, 0.005%, and 0.0075% (w/v), as shown in FIG. 2, while the water temperature was constant at 25°C, as the T1AM concentration increased, the ventilation frequency decreased more rapidly, and the lowest ventilation frequency was 200 minutes at 0.0075% (w/v), 400 minutes at 0.005% (w/v), and 800 minutes at 0.003% (w/v). When the T1AM concentration was 0.002% (w/v), the lowest value was not observed until the end time (1200 minutes) of the experiment and the ventilation frequency was almost not changed when T1AM was not added (0.000% (w/v)).

### Experimental Example 2: Changes in long-term ventilation frequency by appropriate T1AM concentration

### 1) Change in ventilation frequency of Hypostomus plecostomus in 0.005% T1AM solution

In Experimental Example 1, an appropriate T1AM concentration was determined as 0.005% (w/v) by considering a change in ventilation frequency, a survival time, and the like of *Hypostomus plecostomus.* The ventilation frequency of *Hypostomus plecostomus* was measured without the administration of T1AM for 200 minutes while the *Hypostomus plecostomus* was in a tube in a 500 ml solution, and the T1AM concentration was adjusted to be 0.005% (w/v) and then the ventilation frequency of *Hypostomus plecostomus* was measured until 1200 minutes by a unit of 30 minutes.

As a result, as shown in FIG. 3A, an average ventilation frequency of *Hypostomus plecostomus* before the administration of T1AM (-200 to 0 minute) was 180 ± 20 (times/min). After the administration of T1AM, the ventilation frequency reached to the lowest value (10 to 25 times/min) at 400 minutes and then was continued to 1200 minutes which was the end time of the experiment (n = 6).

### 2) Change in ventilation frequency of Hypostomus plecostomus in vehicle solution

To examine the change in ventilation frequency over time, the individual was added in a vehicle solution (-T1AM/+DMSO) and then the ventilation frequency of the individual was measured to 1200 minutes ("1200C").

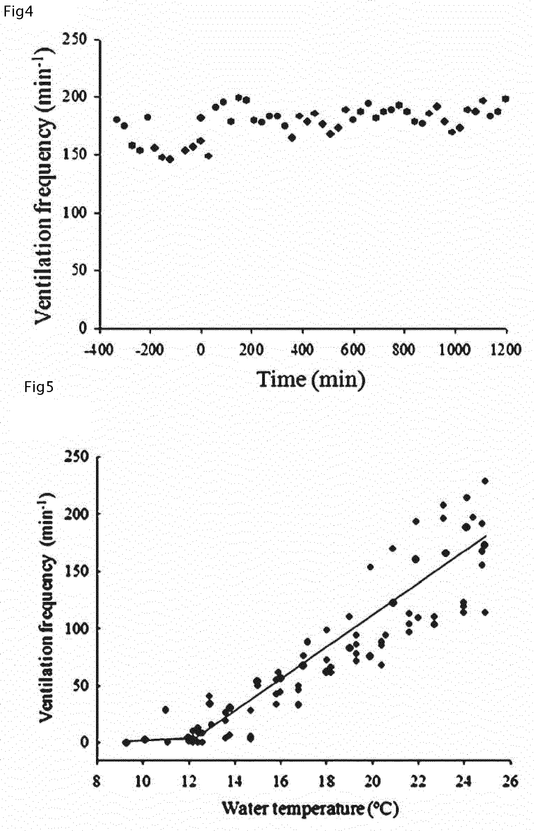

As a result, as shown in FIG. 4, in Experimental Example 1, like the case where T1AM was not added (0.000% (w/v), the ventilation frequency of *Hypostomus plecostomus* was almost not changed.

### 3) Change in ventilation frequency of Hypostomus plecostomus by administration and removal of T1AM

In order to confirm that the ventilation frequency was recovered when the T1AM was removed, T1AM was administered in the same manner as in 1) above, and then a total of 500 ml of the vehicle solution was changed from 600 minutes to 1200 minutes ("1200R") at a speed of 2 ml/min.

As a result, as shown in FIG. 3B, the ventilation frequency of *Hypostomus plecostomus* had the lowest value at about 400 minutes in almost the same manner as the result obtained in 1) above and then rapidly increased after 600 minutes to be recovered to an original level (N = 5).

### Experimental Example 3: Change in ventilation frequency according to water temperature (body temperature)

The ventilation frequency of the individual was measured by cooling the water temperature of the tube (25°C) containing the individual of *Hypostomus plecostomus* to 5°C while lowering the water temperature by 1°C per 30 minutes using a cooling water circulator.

As a result, as shown in FIG. 5, in addition to the decrease in water temperature, the ventilation frequency of *Hypostomus plecostomus* was also decreased proportionally and the water temperature tended to be the lowest ventilation frequency at 12 to 14°C (n = 6).

### Experimental Example 4: Dissolved oxygen consumption rate and survival time according to T1AM administration

After the weight of the individual was measured with an electronic balance, a 0.005% (w/v) T1AM solution was filled in a glass bottle (Z305197, 500 ml, SCHOTT DURAN, DE) and the temperature was maintained in a constant temperature water bath (25°C). After 30 minutes, the individual of *Hypostomus plecostomus* was slowly added, a DO probe was inserted, and then a bottle inlet was closed. Thereafter, the DO value (O₂ mg) and the ventilation frequency of the individual were measured at a constant water temperature every 20 minutes, and the oxygen consumption rate was calculated by dividing the change in dissolved oxygen (DO) in water by the time, and the DO was measured using A Cyberscan PCD 6500 (Eutech instruments, ARC, SG) DO probe (EC620SSP). In the same manner, the DO was measured as a control using a vehicle solution instead of the T1AM solution. As the DO was exhausted over time, the individual stops breathing and loses balance. In this state, when the individual stops breathing for 40 minutes, the individual is regarded as death, and at this time, the time of last breathing is determined to be set the survival time of the individual.

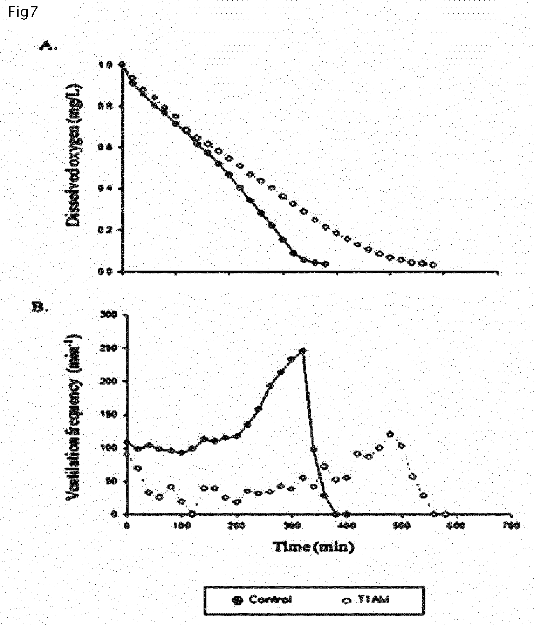

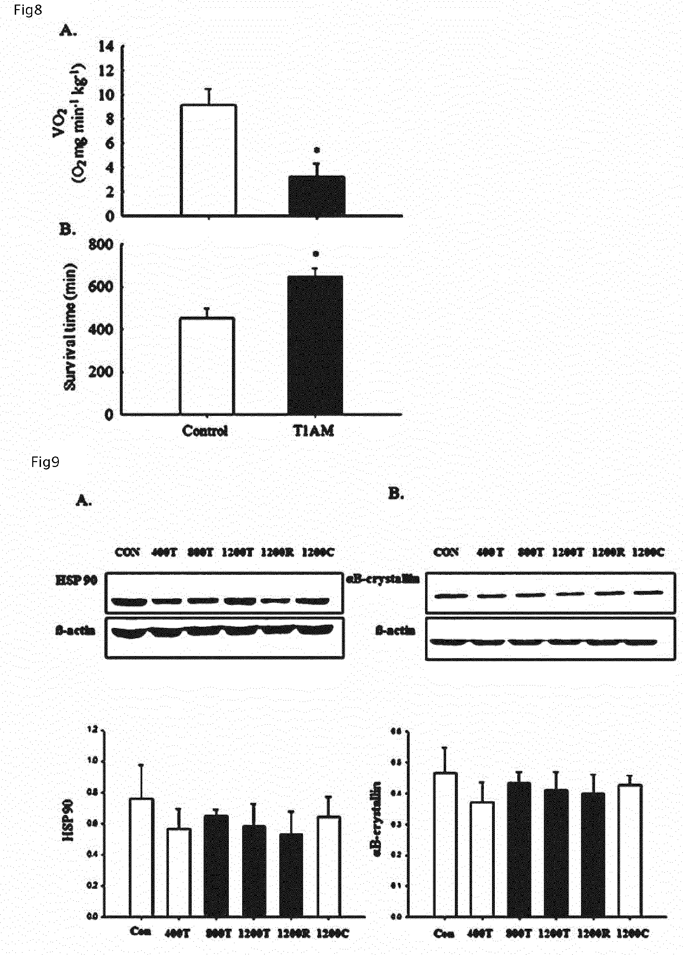

As a result, as shown in FIGS. 7 and 8, it was confirmed that the DO in the T1AM group slowly decreased compared to the control (n = 4; FIG. 7A), and the ventilation frequency in the T1AM group was lower than that of the control and longer continued (N = 4; FIG. 7B). Accordingly, the oxygen consumption rate in the TIAM group was significantly lower than that of the control by 50% or more (n = 4; FIG. 8A). Further, the survival time in the TIAM group was also significantly increased by about 200 minutes compared to the control (n = 4; FIG. 8B).

### Experimental Example 5: Change in expression of chaperone protein

In order to examine whether stress was induced according to induction of long-term hypometabolism, a change in expression of a heat shock protein was analyzed. The heat shock protein is a kind of chaperone molecule and a protein that increases expression in response to induction of stress. For this analysis, brain tissues and other tissues were extracted from each individual of *Hypostomus plecostomus* at 0 min ("Con") before the administration of T1AM, and 400 min (400T), 800 min ("800T"), and 1200 min after administration of T1AM in Experimental Example 2 1), each individual of *Hypostomus plecostomus* ("1200R") in which a total of 500 ml of the vehicle solution was changed at a speed of 2 ml/min from 600 minutes to 1200 minutes after the administration of T1AM in Experimental Example 2 3), and each individual of *Hypostomus plecostomus* ("1200C") measured up to 1200 minutes without administration of T1AM in Experimental Example 2 2), and then frozen in liquid nitrogen (N = 4).

To prepare a whole tissue lysate, the brain tissue was homogenized in a tissue lysis buffer ([20 mM HEPES (pH 7.4), 75 mM NaCl, 2.5 mM MgCl₂, 0.1 mM EDTA, TritonX-100 (0.05% w/v), 20 mM β-glycerophosphate, 1 mM Na₃VO₄, 10 mM NaF, 0.5 mM dithiolthreitol (DTT), a protease inhibitor cocktail tablet (Complete Mini, 11836153001, Mannheim, Roche, GE)]) and then centrifuged at 13,000 rpm for 15 minutes at 4°C to obtain a supernatant. The quantification of the protein was performed using a Bradford assay method and a standard curve was obtained using bovine serum albumin. 1.6 µl of a tissue sample in each group was added in a tube containing 800 µl of sterilized distilled water, added with 200 µl of a Bradford assay reagent (Bio-rad, CA), reacted at room temperature for 10 minutes, and then absorbance (UV-1610PC, Shimadzu, Japan) was measured at λ = 595 nm. The sample was dispensed by 100 µg, rapidly cooled in liquid nitrogen, and stored at - 80°C.

For immunoblot analysis, a 6X sample buffer was added to the whole tissue lysate dispensed by 100 µg, boiled for 5 minutes, and then electrophoresed on an 8% polyacrylamide gel. After electrophoresis, the isolated proteins on the gel were electrotransferred to a nitrocellulose membrane (Amersham, Buckinghamshire, UK) and blotted on the membrane. Then, the blot was confirmed by staining with Ponceau S and the dye was sufficiently removed using 1X TBST and then blocked with a 5% (w/v) skim milk solution. Thereafter, a primary antibody was diluted and added with skim milk and reacted at 4°C for 12 hours. After the reaction, the primary antibody was washed 5 times at 10-min intervals using 1X TBST, and a horseradish peroxidase-labeled secondary antibody which was matched with a host source of the primary antibody reacted at room temperature for 1 hour. After the reaction with the antibody was completed, washing with 1X TBST was repeated and then an ECL detection kit (Amersham) was induced to develop color by an antigen-antibody reaction and exposed to X-ray film to obtain a result. In order to reprobe a loading control, a stripping buffer (Thermo, NMR, IL) was added to the membrane which confirmed the result, reacted in a shaker at room temperature for 15 minutes, washed with 1X TBST five times every 5 minutes, blocked again, and then reacted with the primary antibody. β-actin was used as the loading control and the intensity of the band shown at this time was used as a standard index. In the experiment, the primary antibodies used HSP90 (MBL, SR-B830, JP) and αβ-crystallin (Santa Cruz Biotech, sc-22744, CA), and the secondary antibodies used rabbit IgG of Cell signaling Technology (7074, 3 Trask Lane, MA) and mouse IgG of Bivision (640205, 980 Linda Vista Avenue, Moutain view, Calif.).

As a result, as shown in FIG. 9, the expression of HSP90 (FIG. 8A) and αβ-crystallin (FIG. 8B) did not have a significant difference among all the groups (P> 0.05, n = 4). That is, the expression profile of heat shock protein such as HSP90 and αβ-crystallin was not changed in all situations in which T1AM was administered, not administered, or administered and then removed. Accordingly, it was confirmed that *Hypostomus plecostomus* did not suffer from stress such as oxygen deficiency despite long-term breathing decrease by the hypometabolism-inducing substance, T1AM.

## Claims

1. A method for inducing hypometabolism of fish which is performed by adding the fish into a solution containing a hypometabolism-inducing substance selected from the group consisting of 3-iodothyronamine (T1AM), [D-Ala2,D-Leu5] enkephalin (DADLE), 5'-adenosine monophosphate (5'-AMP), and hydrogen sulfide (H₂S) or administering a hypometabolism-inducing substance selected from the group consisting of 3-iodothyronamine (T1AM), [D-Ala2,D-Leu5] enkephalin (DADLE), 5'-adenosine monophosphate (5'-AMP), and hydrogen sulfide (H₂S) in a solution containing fish.

2. The method for inducing hypometabolism of fish of claim 1, wherein the hypometabolism-inducing substance is 3-iodothyronamine (T1AM).

3. The method for inducing hypometabolism of fish of claim 2, wherein the 3-iodothyronamine (T1AM) is contained at a concentration of 0.002% (w/v) or more in the entire solution.

4. The method for inducing hypometabolism of fish of claim 2, wherein the 3-iodothyronamine (T1AM) is contained at a concentration of 0.002 to 0.01% (w/v) in the entire solution.

5. The method for inducing hypometabolism of fish of claim 1, wherein the hypometabolism of the fish is recovered by removing the hypometabolism-inducing substance.

6. The method for inducing hypometabolism of fish of claim 1, wherein the induction of hypometabolism of the fish is performed under a constant temperature condition.

7. A fish transportation method using a hypometabolism-inducing substance, comprising:
a) adding fish into a solution containing a hypometabolism-inducing substance selected from the group consisting of 3-iodothyronamine (T1AM), [D-Ala2,D-Leu5] enkephalin (DADLE), 5'-adenosine monophosphate (5'-AMP), and hydrogen sulfide (H₂S);
b) moving the fish in the solution in step a) to a destination; and
c) recovering a hypometabolic state of the fish by removing the hypometabolism-inducing substance from the solution.

8. The fish transportation method of claim 1, wherein the hypometabolism-inducing substance is 3-iodothyronamine (T1AM).

9. The fish transportation method using a hypometabolism-inducing substance of claim 8, wherein the 3-iodothyronamine (T1AM) is contained at a concentration of 0.002% (w/v) or more in the entire solution.

10. The fish transportation method using a hypometabolism-inducing substance of claim 8, wherein the 3-iodothyronamine (T1AM) is contained at a concentration of 0.002 to 0.01% (w/v) in the entire solution.

11. The fish transportation method using a hypometabolism-inducing substance of claim 7, wherein the fish transportation is performed under a constant temperature condition.

12. A composition for inducing hypometabolism of fish, containing a hypometabolism-inducing substance selected from the group consisting of 3-iodothyronamine (T1AM), [D-Ala2,D-Leu5] enkephalin (DADLE), 5'-adenosine monophosphate (5'-AMP), and hydrogen sulfide (H₂S); and a solvent.

13. The composition for inducing hypometabolism of fish of claim 12, wherein the hypometabolism-inducing substance is 3-iodothyronamine (T1AM).

14. The composition for inducing hypometabolism of fish of claim 12, wherein the solvent is water or seawater.

15. The composition for inducing hypometabolism of fish of claim 13, wherein the 3-iodothyronamine (T1AM) is contained at a concentration of 0.002% (w/v) or more in the entire solution.

16. The composition for inducing hypometabolism of fish of claim 13, wherein the 3-iodothyronamine (T1AM) is contained at a concentration of 0.002 to 0.01% (w/v) in the entire solution.
